# EUROPEAN PATENT SPECIFICATION

(11) **EP 3 233 125 B1**
(45) Date of publication and mention of the grant of the patent: **03.04.2019**
(21) Application number: 15820454.5
(22) Date of filing: 15.12.2015
(51) Int. Cl.: A61K 47/32, A61K 9/70, A61K 31/381

(54) **A DEVICE FOR THE TRANSDERMAL DELIVERY OF ROTIGOTINE**
VORRICHTUNG ZUR TRANSDERMALEN FREISETZUNG VON ROTIGOTIN
DISPOSITIF POUR L'ADMINISTRATION TRANSDERMIQUE DE ROTIGOTINE

(30) Priority: 16.12.2014 AR P140104682
(43) Date of publication of application: 25.10.2017
(73) Proprietor: Amarin Technologies S.A., Ciudad Autónoma de Buenos Aires 1416 (AR)
(72) Inventor: SCASSO, Alejandro Fabio, Buenos Aires 1852 (AR); STEFANO, Francisco José Evaristo, Ciudad Autónoma de Buenos Aires 1425 (AR)
(74) Representative: Mewburn Ellis LLP
(86) International application number: PCT/EP2015/079770
(87) International publication number: WO 2016/096840

(56) References cited:
- WO-A1-2013/075822
- WO-A1-2014/079573
- WO-A1-2014/181840
- JUSTIN R HUGHEY ET AL: "Thermal processing of a poorly water-soluble drug substance exhibiting a high melting point: The utility of KinetiSolDispersing", INTERNATIONAL JOURNAL OF PHARMACEUTICS, ELSEVIER BV, NL, vol. 419, no. 1, 8 August 2011 (2011-08-08), pages 222-230, XP028306633, ISSN: 0378-5173, DOI: 10.1016/J.IJPHARM.2011.08.007 [retrieved on 2011-08-16]
- BASF: "Soluplus -Technical information", INTERNET CITATION, July 2010 (2010-07), pages 1-8, XP002666808, Retrieved from the Internet: URL:https://industries.basf.com/en/documen tDownload.8805242743253.Soluplus® - Technical Information.pdf [retrieved on 2012-01-10]

## Description

The present invention refers to a Transdermal Delivery System (TDS) for the transdermal administration of rotigotine, comprising a layer of adhesive matrix, a film backing and a release liner film, where the layer of adhesive matrix comprises Rotigotine, an adhesive polymer selected among silicone adhesives, acrylic adhesives, polyisobutylene adhesives and adhesive block polymers of the type of Styrene-Isoprene-Styrene or Styrene-Butadiene-Styrene or a mixture of those adhesive polymers, an antioxidant and a polyethylene glycol, polyvinyl caprolactam and polyvinyl acetate copolymer.

Preferably, the adhesive block polymer is of the Styrene-Isoprene-Styrene type and the antioxidant is ascorbyl palmitate.

### BACKGROUND OF THE INVENTION

The transdermal administration of drugs has major advantages in respect of other routes of administration. Among some of these advantages, there are included its comfort, the possibility of releasing the therapeutically active substance in a controlled and predictable manner, as well as its feasibility to promptly interrupt the administration of the released drug, in case of adverse reactions (by peeling off the device from the skin). It also allows to improve the achievement of therapeutics schemes and to reduce some of the adverse effects related to the oral therapy of a particular drug.

By means of transdermal delivery systems it would be possible the systemic administration of different drugs through the skin. The first ones to be approved by the Food and Drug Administration (FDA) were the devices containing the drug Scopolamine, aimed to the prevention and relief of motion sickness. Years later, other transdermal devices for the treatment of different pathologies were approved, including devices for the transdermal administration of hormones, analgesics, non-steroidal antiinflammatories, nitroglycerine and fentanyl.

Although the above-mentioned devices introduce many advantages, not all drugs have been able to be incorporated successfully into this kind of devices for their transdermal administration.

For example, devices for transdermal release of steroid hormones denote inconvenients in their physical stability, therefore, several method have been proposed (see patents US 6.465.005 and US 5.676.968) to avoid the crystallisation of the active substances.

Rotigotine is a drug indicated for the treatment of Parkinson's disease and restless legs syndrome. The product Neupro® is a device for the transdermal administration of rotigotine, approved in U.S.A. as well as in Europe.

The patents US 6.884.434 and US 7.413.747 describe a device for the transdermal administration of rotigotine whose adhesive matrix contains a silicone or polyacrylate adhesive polymer and polyvinylpyrrolidone as crystallisation inhibitor of the drug.

However, It has been determined that the product Neupro®, which contains a silicone adhesive polymer and polyvinylpyrrolidone as part of its composition, denotes physical stability problems, and it is observed crystallisation of rotigotine in the short term if it is not kept in a refrigerated environment. The patent US 8.232.414 refers to a crystallized form of rotigotine (polymorph II), thermodynamically more stable than the previously known one (polymorph I), and which is the one form that crystallizes in Neupro® patches (see "Comparison of the bioavailability and adhesiveness of different rotigotine transdermal patch formulations," Current Medical Research & Opinion. 29 (2013)1657-62).

The PCT patent applications WO 2013075822 A1 and WO 2013075823 A1 refer to transdermal administration systems containing Rotigotine, into an adhesive matrix composed by a Styrene-Butadiene block copolymer. In the applications it is proposed as the preferred formulation which contains copovidone as crystallisation inhibitor of the drug.

### BRIEF DESCRIPTION OF THE INVENTION

The present invention refers to a transdermal administration system (TDS) for the transdermal administration of rotigotine, which comprises an adhesive matrix layer, a backing film and a release layer, where the adhesive matrix contains the rotigotine, an adhesive polymer and a copolymer of polyethylene glycol, polyvinylcaprolactam and polyvinylacetate. Preferably, the adhesive block polymer is from the type of styrene-isoprene-styrene (SIS).

The device described according to the present invention is physically as well as chemically stable and has good adhesive properties, as well as appropriate pharmacokinetic parameters.

### FULL DESCRIPTION OF THE INVENTION

The inventors of the present invention have found that, surprisingly, the crystallization of the formulated rotigotine in a TDS (in therapeutically effective amounts) could be avoided if such TDS comprises an adhesive matrix that, in turn, comprises the rotigotine, an adhesive polymer and a copolymer of Polyethylene glycol, polyvinylcaprolactam and polyvinylacetate.

It is common knowledge for experts in the technique that, when formulating a TDS, a drug concentration as high as possible is desirable, so as to provide with a good driving force for the drug permeation through the skin. However, a too high drug concentration may generate crystals formation, if it is solid at storage temperature. The drug crystallisation may be harmful for the proper performance of the TDS due to the fact that it can impact on its permeation through the skin, as well as the adhesive properties of the product. For this reason, the avoidance of crystals formation during the storage of the product is essential to ensure its proper performance during its shelf life.

It is also preferred that a TDS shows good adhesive properties for its correct performance. There are different types of adhesive polymers which are usually used in TDS formulations and that could be used to produce formulations within the range of the present invention. Preferably, TDS among the range of this invention have, at least, an adhesive polymer selected from the group of silicon adhesives, acrylic adhesives, polyisobutylene adhesives and styrene-isoprene-styrene or styrene-butadiene-styrene block copolymers. In another preferred realization, the device of the invention has a mixture of the above mentioned adhesives.

The inventors of this device have found that the physical stability of rotigotine patches is unsatisfactory when they are formulated using adhesive matrix simply composed by an adhesive polymer or by a mixture of adhesive polymers of the types mentioned above. In formulations of this kind, it is observed crystallization of the drug during the first month of storage, in long term stability conditions (25°C, 60% relative humidity) or accelerated stability (40°C, 75% relative humidity).

Crystallisation inhibitors recognized by experts in the technique, such as polyvinylpyrrolidone, are not effective for utilization in the realizations of the present invention. The commercial product Neupro® contains polyvinylpyrrolidone as part of its formulation, in a matrix that also comprises a silicone adhesive, and this compound fails to prevent the crystallization of the drug.

On the other hand, the polyvinylpyrrolidone does not properly incorporate to a hydrophobic matrix composed by styrene-isoprene-styrene or styrene-butadiene-styrene copolymers.

In patent applications WO 2013075822 A1 and WO 2013075823 A1, the use of copovidone is recommended as crystallization inhibitor, structurally similar to polyvinylpyrrolidone.

The inventors of the present invention found, surprisingly, that a Polyethylene glycol, Polyvinylcaprolatam and Polyvinylacetate copolymer incorporated to the matrix provides a stable system which does not experience crystallization from Rotigotine, that has excellent adhesive properties and has a skin permeation similar to the reference product Neupro®.

TDSs comprised within the scope of this invention comprise an adhesive matrix with an adhesive polymer selected from the group of silicone adhesives, acrylic adhesives, adhesives from polyisobutylene and styrene-Isoprene-Styrene or styrene-butadiene-styrene block copolymers. This copolymer can be present in the adhesive matrix in a concentration between 60.0% and 90.0%. Preferably, the copolymer has a concentration between 70.0% and 80.0%

Examples of silicone adhesives that can be used in formulations within the range of this invention are Bio-PSA® 7-4301, Bio-PSA® 7-4302 and Bio-PSA® 7-4602, commercialized by Dow Corning®. Examples of acrylic adhesives that can be used in formulations within the range of this invention are Duro-Tak® 87-2287, Duro-Tak® 87-4287 (both with hydroxyl functionality), Duro-Tak® 87-2353 (with carboxyl functionality) and Duro-Tak® 87-4098 (no functionality), commercialized by Henkel®. An example of polyisobutylene that can be used in formulations within the range of this invention is Duro-Tak 87-608A®, commercialized by Henkel®. Examples of Styrene-Isoprene-Styrene or Styrene-Butadiene-Styrene block copolymers which can be used in formulations within the range of this invention are Duro-Tak® 87-6911, commercialized by Henkel® and Roderm® MD-153, commercialized by The Dow Chemical Company®, both styrene-isoprene-styrene copolymers.

The rotigotine shall be found in the adhesive matrix, in a concentration sufficient to provide a suitable therapeutic effect. As an example, the rotigotine could be found in the adhesive matrix in a concentration between 5.0% and 30.0%. Preferably, rotigotine could be found in a concentration between 7.5% and 13.0%.

The polyethylene glycol, polyvinyl caprolactam and polyvinyl acetate copolymer can be composed by different proportions of each of the monomers that are composed of. Preferably, the copolymer contains polyethylene glycol, polyvinyl caprolactam and polyvinyl acetate in a proportion of 13/57/30. In a particularly preferred composition, the copolymer is the product commercialized by BASF® under the commercial name Soluplus®. The copolymer can be found in the adhesive matrix in a concentration between 1.0% and 10.0%. Preferably, the copolymer is found in the adhesive matrix in a concentration between 1.0% and 5.0%.

The TDSs comprised within the scope of this invention can also comprise as part of the composition of the adhesive matrix, several permeation enhancers which are well known by the experts in the art. Examples of permeation enhancers that can be included in the implementation of the present invention are: organic acids such as stearic, isoestearic, oleic, linoleic, linolenic, levulinic and other acids; fatty acid esters such as isopropyl myristate, oleyl oleate and others; other liquid compounds such as N-methyl-2-pyrrolidone, dimethyl sulfoxide and dimethyl isosorbide, among others.

Preferably, the formulation includes levulinic acid that, apart from the fact of being an enhancer, it also operates as co-solvent in the matrix, facilitating the dissolution of rotigotine. A composition of the present invention, which is particularly preferred, includes levulinic acid in a concentration between 3.0% and 7.0%. An even more preferred implementation of the present invention includes, together with levulinic acid in a concentration between 3.0% and 7.0%, oleyl oleate in a concentration between 3.0% and 7.0%.

Rotigotine is a drug sensitive to the oxidative degradation. An expert may minimize or avoid that degradation by means of the addition of one or more antioxidants to the adhesive matrix of the TDS. Some characteristic antioxidants that can be used in implementations of the present invention are Ascorbic acid, Ascorbyl palmitate, α-Tocopherol, Butylated hydroxytoluene and Butylated hydroxyanisole, although the list is not exhaustive. Preferably, the adhesive matrix of the TDS includes Ascorbyl palmitate.

In addition to an adhesive matrix, an expert in the art will understand that the TDSs comprised within the range of this invention comprise a backing and a release liner. Non limiting examples of backings that can be used in implementations of the present invention are: Polythene layers, Polyolefin layers, Ethyl acetate and vinyl layers polyester layers, among others. Furthermore, silicone polyester layers and Teflon polyester layers can be used as release layers.

A particularly preferred implementation of the present invention is a TDS that comprises a layer of adhesive matrix, a backing and a release liner where the adhesive matrix comprise rotigotine in a concentration of 10.0%, a block adhesive polymer of the Styrene-Isoprene-Styrene type in a concentration of 77.0%, Levulinic acid in a concentration of 5.0%, Oleyl oleate in a concentration of 5.0%, Ascorbyl palmitate in a concentration of 0.5% and Soluplus® in a concentration of 2.5%.

Unless otherwise expressly provided, all percentages indicated herein are expressed as percentages weight in weight.

### EXAMPLES OF IMPLEMENTATION

### Example 1

### Evaluation of the physical stability of different compositions

| **Lot** | **Composition** | | **Physical Stability after 2 months of storage** |
|---|---|---|---|
| 1 | Duro-Tak® (DT) 87-6911 | 90.0% | Crystals are observed after 14 days of storage at 25 or 40 °C |
| | Rotigotine base | 10.0% | |
| 2 | Rotigotine base | 11.1% | Crystals are observed after a month of storage at 25 or 40 °C |
| | DT 87-6911 | 72.5% | |
| | Di-methyl-isosorbide | 13.9% | |
| | N-methyl-2-pyrrolidone | 2.5% | |
| 3 | Rotigotine base | 11.1% | Discarded; as Polyvinylpyrrolidone does not incorporate to the adhesive matrix |
| | DT 87-6911 | 61.4% | |
| | Di-methyl-isosorbide | 13.9% | |
| | N-methyl-2-pyrrolidone | 2.5% | |
| | Polyvinylpyrrolidone | 11.1% | |
| 4 | Rotigotine base | 11.1% | Crystals formation was not observed at 25 or 40 °C |
| | DT 87-6911 | 61.4% | |
| | Di-methyl-isosorbide | 13.9% | |
| | N-methyl-2-pyrrolidone | 2.5% | |
| | Soluplus® | 11.1% | |
| 5 | Rotigotine base | 15.8% | Crystals formation was not observed at 25 or 40 °C |
| | DT 87-6911 | 65.9% | |
| | 2NMP | 5.0% | |
| | Soluplus® | 12.7% | |
| | Ascorbyl palmitate | 0.6% | |
| 6 | Rotigotine base | 19.0% | Crystals formation was not observed at 25 or 40 °C |
| | DT 87-6911 | 62.7% | |
| | 2NMP | 5.0% | |
| | Soluplus® | 12.7% | |
| | Ascorbyl palmitate | 0.6% | |
| 7 | Rotigotine base | 22.2% | Crystals formation was not observed at 25 or 40 °C |
| | DT 87-6911 | 59.5% | |
| | 2NMP | 5.0% | |
| | Soluplus® | 12.7% | |
| | Ascorbyl palmitate | 0.6% | |
| 8 | Rotigotine base | 25.3% | Crystals formation was not observed at 25 or 40 °C |
| | DT 87-6911 | 56.4% | |
| | 2NMP | 5.0% | |
| | Soluplus® | 12.7% | |
| | Ascorbyl palmitate | 0.6% | |
| 9 | Rotigotine base | 12.7% | Crystals formation was not observed at 25 or 40 °C |
| | DT 87-6911 | 78.5% | |
| | 2NMP | 5.0% | |
| | Soluplus® | 3.2% | |
| | Ascorbyl palmitate | 0.6% | |
| 10 | Rotigotine base | 12.7% | Crystals formation was not observed at 25 or 40 °C |
| | DT 87-6911 | 75.4% | |
| | 2NMP | 5.0% | |
| | Soluplus® | 6.3% | |
| | Ascorbyl palmitate | 0.6% | |
| 11 | Rotigotine base | 12.7% | Crystals formation was not observed at 25 or 40 °C |
| | DT 87-6911 | 72.2% | |
| | 2NMP | 5.0% | |
| | Soluplus® | 9.5% | |
| | Ascorbyl palmitate | 0.6% | |
| 12 | Rotigotine base | 12.7% | Crystals formation was not observed at 25 or 40 °C |
| | DT 87-6911 | 62.7% | |
| | 2NMP | 5.0% | |
| | Soluplus® | 19.0% | |
| | Ascorbyl palmitate | 0.6% | |
| 13 | Rotigotine base | 12.7% | Crystals formation was not observed at 25 or 40 °C |
| | DT 87-6911 | 56.4% | |
| | 2NMP | 5.0% | |
| | Soluplus® | 25.3% | |
| | Ascorbyl palmitate | 0.6% | |
| 14 | Rotigotine base | 11.4% | Crystals formation was not observed at 25 or 40 °C |
| | DT 87-6911 | 73.6% | |
| | 2NMP | 3.0% | |
| | Soluplus® | 11.4% | |
| | Ascorbyl palmitate | 0.6% | |
| 15 | Rotigotine base | 12.0% | Crystals formation was not observed at 25 or 40 °C |
| | DT 87-6911 | 71.4% | |
| | 2NMP | 4.0% | |
| | Soluplus® | 12.0% | |
| | Ascorbyl palmitate | 0.6% | |
| 16 | Rotigotine base | 13.4% | Crystals formation was not observed at 25 or 40 °C |
| | DT 87-6911 | 66.5% | |
| | 2NMP | 6.0% | |
| | Soluplus® | 13.4% | |
| | Ascorbyl palmitate | 0.7% | |
| 17 | Rotigotine base | 10.0% | Crystals formation was not observed at 25 or 40 °C |
| | Bio-PSA 7-4302 | 79.5% | |
| | Soluplus® | 10.0% | |
| | Ascorbyl palmitate | 0.50% | |
| 18 | Rotigotine base | 10.0 | Crystals formation was not observed at 25 or 40 °C |
| | DT 87-2287 | 79.5 | |
| | Soluplus® | 10.0 | |
| | Ascorbyl palmitate | 0.50 | |
| 19 | Rotigotine base | 25.0 | Crystals formation was not observed at 25 or 40 °C |
| | DT 87-2287 | 64.5 | |
| | Soluplus® | 10.0 | |
| | Ascorbyl palmitate | 0.50 | |
| 20 | Rotigotine base | 10.0 | Crystals formation was not observed at 25 or 40 °C |
| | DT 87-4287 | 79.5 | |
| | Soluplus® | 10.0 | |
| | Ascorbyl palmitate | 0.50 | |
| 21 | Rotigotine base | 25.0 | Crystals formation was not observed at 25 or 40 °C |
| | DT 87-4287 | 64.5 | |
| | Soluplus® | 10.0 | |
| | Ascorbyl palmitate | 0.50 | |
| 22 | Rotigotine base | 10.0 | Crystals formation was not observed at 25 or 40 °C |
| | DT 87-2353 | 79.5 | |
| | Soluplus® | 10.0 | |
| | Ascorbyl palmitate | 0.50 | |
| 23 | Rotigotine base | 10.0 | Crystals formation was not observed at 25 or 40 °C |
| | DT 87-4098 | 79.5 | |
| | Soluplus® | 10.0 | |
| | Ascorbyl palmitate | 0.5 | |
| 24 | Rotigotine base | 10.0 | Crystals formation was not observed at 25 or 40 °C |
| | DT 87-6911 | 84.5 | |
| | Soluplus® | 5.0 | |
| | Ascorbyl palmitate | 0.5 | |
| 25 | Rotigotine base | 10.0 | Crystals formation was not observed at 25 or 40 °C |
| | DT 87-6911 | 82.0 | |
| | Soluplus® | 7.5 | |
| | Ascorbyl palmitate | 0.5 | |
| 26 | Rotigotine base | 10.0 | Crystals formation was not observed at 25 or 40 °C |
| | DT 87-6911 | 79.5 | |
| | Soluplus® | 10.0 | |
| | Ascorbyl palmitate | 0.5 | |
| 27 | Rotigotine base | 10.0 | Crystals formation was not observed at 25 or 40 °C |
| | DT 87-6911 | 82.0 | |
| | Soluplus® | 2.5 | |
| | Oleyl oleate | 5.0 | |
| | Ascorbyl palmitate | 0.5 | |
| 28 | Rotigotine base | 10.0 | Crystals formation was not observed at 25 or 40 °C |
| | DT 87-6911 | 82.0 | |
| | Soluplus® | 2.5 | |
| | Levulinic acid | 5.0 | |
| | Ascorbyl palmitate | 0.5 | |
| 29 | Rotigotine base | 10.0 | Crystals formation was not observed at 25 or 40 °C |
| | DT 87-6911 | 77.0 | |
| | Soluplus® | 2.5 | |
| | Oleyl oleate | 5.0 | |
| | Levulinic acid | 5.0 | |
| | Ascorbyl palmitate | 0.5 | |

It is observed in the table that the incorporation of Soluplus® allows the obtention of a physically stable TDS.

### Example 2

### Evaluation of chemical stability in a preferred composition

It was evaluated the chemical stability of a preferred composition of the present invention, by determination of the quantity of impurities determined after 3 months of storage at 40 °C and a relative humidity of 75% by means of an appropriate HPLC technique.

The quantity of impurities generated during the storage is shown as a percentage weight by weight (%p/p) related to the quantity of Rotigotine.

| **Lot** | **Composition** | | **Generated Impurities** | |
|---|---|---|---|---|
| | | | **Impurity** | **% p/p** |
| 29 | Rotigotine base | 10.0 | | |
| | DT 87-6911 | 77.0 | | |
| | Soluplus | 2.5 | Impurity 1 | 0.3 |
| | Oleyl oleate | 5.0 | Impurity2 | ≤ 0.1 |
| | Levulinic acid | 5.0 | Totals | 0.3 |
| | Ascorbyl palmitate | 0.5 | | |

In the table it can be observed that the generation of impurities is significantly reduced.

### Example 3

### Comparative permeation as regards Neupro®

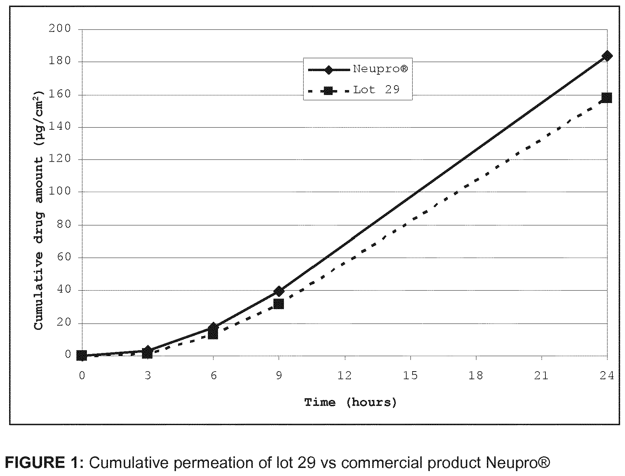

An *in vitro* permeation experiment was conducted through the skin, comparing the performance of the commercial product Neupro® with Lot 29. As permeation model, intact human skin was used. The results obtained are shown in the following table and in Figure 1.

| **Time (h)** | **Cumulative amount of Rotigotine (µg/cm²)** | |
|---|---|---|
| | **Neupro**® | **Lot 29** |
| 0 | 0.0 | 0.0 |
| 3 | 3.3 | 1.5 |
| 6 | 17.4 | 13.2 |
| 9 | 39.6 | 31.3 |
| 24 | 184.2 | 157.9 |

## Claims

1. A device for the transdermal administration of rotigotine that comprises an adhesive matrix layer, a backing film and a release liner, **characterized in that** said adhesive matrix comprises:
rotigotine;
an adhesive polymer selected from the group consisting of silicone adhesives, acrylic adhesives, poly-isobutylene adhesives and styrene- isoprene-styrene or styrene- butadiene-styrene block copolymers or a mixture thereof;
a copolymer of polyethylene glycol, polyvinyl caprolactam and polyvinyl acetate; and
optionally levulinic acid.

2. A device for the transdermal administration of rotigotine, according to claim 1, **characterized in that** said adhesive matrix comprises rotigotine, an adhesive of styrene-isoprene-styrene block copolymer and a polyethylene glycol, polyvinyl caprolactam and polyvinyl acetate copolymer.

3. A device for the transdermal administration of rotigotine, according to the preceding claim, **characterized in that** the concentration of said styrene- isoprene-styrene block copolymer is from about 60 w/w% to about 90 w/w%.

4. A device for the transdermal administration of rotigotine, according to the preceding claim, **characterized in that** the concentration of said styrene-isoprene-styrene block polymer is from about 70 w/w% to about 80 w/w%.

5. A device for the transdermal administration of rotigotine according to any one of the preceding claims, **characterized in that** said copolymer of polyethylene glycol, polyvinyl caprolactam and polyvinyl acetate contains polyethylene glycol, polyvinyl caprolactam and polyvinyl acetate in a ratio of 13/57/30.

6. A device for the transdermal administration of rotigotine according to the preceding claim, **characterized in that** the concentration of said copolymer of polyethylene glycol, polyvinyl caprolactam and polyvinyl acetate is from about 1.0 w/w% to about 10.0 w/w%.

7. A device for the transdermal administration of rotigotine according to the preceding claim, **characterized in that** the concentration of said coplymer of polyethylene glycol, polyvinyl caprolactam and polyvinyl acetate is from about 1.0 w/w% to about 5.0 w/w%.

8. A device for the transdermal administration of rotigotine according to any one of the preceding claims, **characterized in that** the concentration of said rotigotine is from about 5.0 w/w% to about 30.0 w/w%.

9. A device for the transdermal administration of rotigotine according to the preceding claim, **characterized in that** the concentration of said rotigotine is from about 7.5 w/w% to about 13.0 w/w%.

10. A device for the transdermal administration of rotigotine according to any of the preceding claims, where the levulinic acid is found in a concentration of between 3.0 w/w% and 7.0 w/w%.

11. A device for the transdermal administration of rotigotine, according to any one of the preceding claims, **characterized by** further comprising an antioxidant and an enhancer.

12. A device for the transdermal administration of rotigotine, according to the preceding claim, **characterized in that** the enhancer is selected from the group consisting in isopropyl myristate, isostearic acid, oleic acid, oleyl oleate, lauric alcohol, dodecanol, N-methyl-2-Pyrrolidone, di-methyl-sulfoxide and di-methyl-isosorbide, and **in that** said enhancer in the adhesive matrix is present in a concentration from about 2 w/w% to about 20 w/w%.

13. A device for the transdermal administration of rotigotine, according to the preceding claim, **characterized in that** the enhancer is oleyl oleate, which is present at a concentration from about 3.0 to about 7.0 w/w%.

14. A device for the transdermal administration of rotigotine, according to any one of claims 11 to 13 **characterized in that** the antioxidant is ascorbyl palmitate.

## Patentansprüche

1. Vorrichtung zur transdermalen Verabreichung von Rotigotin, die eine Klebstoffmatrixschicht, einen Trägerfilm und eine Abziehfolie umfasst, **dadurch gekennzeichnet, dass** diese Klebstoffmatrix Folgendes umfasst:
Rotigotin;
ein Klebstoffpolymer, das aus der Gruppe, bestehend aus Silikonklebstoffen, Acrylklebstoffen, Polyisobutylenklebstoffen und Styrol-Isopren-Styrol- oder StyrolButadien-Styrol-Blockcopolymeren und einem Gemisch davon ausgewählt ist;
ein Copolymer von Polyethylenglykol, Polyvinylcaprolactam und Polyvinylacetat; und
gegebenenfalls Lävulinsäure.

2. Vorrichtung zur transdermalen Verabreichung von Rotigotin nach Anspruch 1, **dadurch gekennzeichnet, dass** die Klebstoffmatrix Rotigotin, einen Klebstoff aus einem Styrol-Isopren-Styrol-Blockcopolymer und ein Polyethylenglykol-, Polyvinylcaprolactam- und Polyvinylacetat-Copolymer umfasst.

3. Vorrichtung zur transdermalen Verabreichung von Rotigotin nach dem vorangegangenen Anspruch, **dadurch gekennzeichnet, dass** die Konzentration des Styrol-Isopren-Styrol-Blockcopolymers etwa 60 Gew.-% bis etwa 90 Gew.-% beträgt.

4. Vorrichtung zur transdermalen Verabreichung von Rotigotin nach dem vorangegangenen Anspruch, **dadurch gekennzeichnet, dass** die Konzentration des Styrol-Isopren-Styrol-Blockcopolymers etwa 70 Gew.-% bis etwa 80 Gew.-% beträgt.

5. Vorrichtung zur transdermalen Verabreichung von Rotigotin nach einem der vorangegangenen Ansprüche, **dadurch gekennzeichnet, dass** das Copolymer von Polyethylenglykol, Polyvinylcaprolactam und Polyvinylacetat Polyethylenglykol, Polyvinylcaprolactam und Polyvinylacetat in einem Verhältnis von 13/57/30 enthält.

6. Vorrichtung zur transdermalen Verabreichung von Rotigotin nach dem vorangegangenen Anspruch, **dadurch gekennzeichnet, dass** die Konzentration des Copolymers von Polyethylenglykol, Polyvinylcaprolactam und Polyvinylacetat etwa 1,0 Gew.-% bis etwa 10,0 Gew.-% beträgt.

7. Vorrichtung zur transdermalen Verabreichung von Rotigotin nach dem vorangegangenen Anspruch, **dadurch gekennzeichnet, dass** die Konzentration des Copolymers von Polyethylenglykol, Polyvinylcaprolactam und Polyvinylacetat etwa 1,0 Gew.-% bis etwa 5,0 Gew.-% beträgt.

8. Vorrichtung zur transdermalen Verabreichung von Rotigotin nach einem der vorangegangenen Ansprüche, **dadurch gekennzeichnet, dass** die Konzentration von Rotigotin etwa 5,0 Gew.-% bis etwa 30,0 Gew.-% beträgt.

9. Vorrichtung zur transdermalen Verabreichung von Rotigotin nach dem vorangegangenen Anspruch, **dadurch gekennzeichnet, dass** die Konzentration von Rotigotin etwa 7,5 Gew.-% bis etwa 13,0 Gew.-% beträgt.

10. Vorrichtung zur transdermalen Verabreichung von Rotigotin nach einem der vorangegangenen Ansprüche, wobei die Lävulinsäure in einer Konzentration zwischen 3,0 Gew.-% und 7,0 Gew.-% vorliegt.

11. Vorrichtung zur transdermalen Verabreichung von Rotigotin nach einem der vorangegangenen Ansprüche, **dadurch gekennzeichnet, dass** sie außerdem ein Antioxidans und einen Verstärker umfasst.

12. Vorrichtung zur transdermalen Verabreichung von Rotigotin nach dem vorangegangenen Anspruch, **dadurch gekennzeichnet, dass** der Verstärker aus der aus Isopropylmyristat, Isostearinsäure, Ölsäure, Oleyloleat, Laurinalkohol, Dodecanol, N-Methyl-2-pyrrolidon, Dimethylsulfoxid und Dimethylisosorbid bestehenden Gruppe ausgewählt ist und dass der Verstärker in der Klebstoffmatrix in einer Konzentration von etwa 2 Gew.-% bis etwa 20 Gew.-% vorliegt.

13. Vorrichtung zur transdermalen Verabreichung von Rotigotin nach dem vorangegangenen Anspruch, **dadurch gekennzeichnet, dass** der Verstärker Oleyloleat ist, das in einer Konzentration von etwa 3,0 bis etwa 7,0 Gew.-% vorliegt.

14. Vorrichtung zur transdermalen Verabreichung von Rotigotin nach einem der Ansprüche 11 bis 13, **dadurch gekennzeichnet, dass** das Antioxidans Ascorbylpalmitat ist.

## Revendications

1. Dispositif pour l'administration transdermique de rotigotine qui comporte une couche de matrice adhésive, un film de renfort et une doublure antiadhésive, **caractérisé en ce que** ladite matrice adhésive comprend :
la rotigotine ;
un polymère adhésif choisi dans le groupe comprenant des adhésifs de silicone, des adhésifs acryliques, des adhésifs de polyisobutylène et des copolymères séquencés de styrène-isoprène-styrène ou styrène-butadiène-styrène, ou un mélange de ceux-ci ;
un copolymère de polyéthylène glycol, de polyvinylcaprolactame et d'acétate de polyvinyle ; et
facultativement de l'acide lévulinique.

2. Dispositif pour l'administration transdermique de rotigotine selon la revendication 1, **caractérisé en ce que** ladite matrice adhésive comprend de la rotigotine, un adhésif de copolymère séquencé de styrène-isoprène-styrène et un copolymère de polyéthylène glycol, de polyvinylcaprolactame et d'acétate de polyvinyle.

3. Dispositif pour l'administration transdermique de rotigotine selon la revendication précédente, **caractérisé en ce que** la concentration dudit copolymère séquencé de styrène-isoprène-styrène est d'environ 60 % poids/poids à environ 90 % poids/poids.

4. Dispositif pour l'administration transdermique de rotigotine selon la revendication précédente, **caractérisé en ce que** la concentration dudit polymère séquencé de styrène-isoprène-styrène est d'environ 70 % poids/poids à environ 80 % poids/poids.

5. Dispositif pour l'administration transdermique de rotigotine selon l'une quelconque des revendications précédentes, **caractérisé en ce que** ledit copolymère de polyéthylène glycol, de polyvinylcaprolactame et d'acétate de polyvinyle contient du polyéthylène glycol, du polyvinylcaprolactame et de l'acétate de polyvinyle selon un rapport de 13/57/30

6. Dispositif pour l'administration transdermique de rotigotine selon la revendication précédente, **caractérisé en ce que** la concentration dudit copolymère de polyéthylène glycol, de polyvinylcaprolactame et d'acétate de polyvinyle est d'environ 1,0 % poids/poids à environ 10,0 % poids/poids.

7. Dispositif pour l'administration transdermique de rotigotine selon la revendication précédente, **caractérisé en ce que** la concentration dudit copolymère de polyéthylène glycol, de polyvinylcaprolactame et d'acétate de polyvinyle est d'environ 1,0 % poids/poids à environ 5,0 % poids/poids.

8. Dispositif pour l'administration transdermique de rotigotine selon l'une quelconque des revendications précédentes, **caractérisé en ce que** la concentration de ladite rotigotine est d'environ 5,0 % poids/poids à environ 30,0 % poids/poids.

9. Dispositif pour l'administration transdermique de rotigotine selon la revendication précédente, **caractérisé en ce que** la concentration de ladite rotigotine est d'environ 7,5 % poids/poids à environ 13,0 % poids/poids.

10. Dispositif pour l'administration transdermique de rotigotine selon l'une quelconque des revendications précédentes, dans lequel l'acide lévulinique se trouve à une concentration comprise entre 3,0 % poids/poids et 7,0 % poids/poids.

11. Dispositif pour l'administration transdermique de rotigotine selon l'une quelconque des revendications précédentes, **caractérisé en ce qu'**il comprend en outre un antioxydant et un amplificateur.

12. Dispositif pour l'administration transdermique de rotigotine selon la revendication précédente, **caractérisé en ce que** l'amplificateur est choisi dans le groupe constitué par le myristate d'isopropyle, l'acide isostéarique, l'acide oléique, l'oléate d'oléyle, l'alcool laurique, le dodécanol, la n-méthyl-2-pyrrolidone, le diméthylsulfoxyde et le diméthylisosorbide, et **en ce que** ledit amplificateur dans la matrice adhésive est présente à une concentration d'environ 2 % poids/poids à environ 20 % poids/poids.

13. Dispositif pour l'administration transdermique de rotigotine selon la revendication précédente, **caractérisé en ce que** l'amplificateur est l'oléate d'oléyle, qui est présent à une concentration d'environ 3,0 à environ 7,0 % poids/poids.

14. Dispositif pour l'administration transdermique de rotigotine selon l'une quelconque des revendications 11 à 13, **caractérisé en ce que** l'antioxydant est le palmitate d'ascorbyle.
